(19) Europäisches Patentamt

European Patent Office

Office européen des brevets

(11) **EP 1 261 556 B1**

(12) **EUROPEAN PATENT SPECIFICATION**

(45) Date of publication and mention
of the grant of the patent:
**25.05.2005 Bulletin 2005/21**

(51) Int Cl.[7]: **C03C 17/23**, C03C 21/00

(86) International application number:
**PCT/FI2001/000188**

(21) Application number: **01913903.9**

(22) Date of filing: **23.02.2001**

(87) International publication number:
**WO 2001/066479 (13.09.2001 Gazette 2001/37)**

(54) **A METHOD FOR ETCHING THE SURFACE OF A BIOACTIVE GLASS**

VERFAHREN ZUR ÄTZUNG DER OBERFLÄCHE EINES BIOAKTIVEN GLASSES

PROCEDE PERMETTANT DE GRAVER LA SURFACE D'UN VERRE BIOACTIF

(84) Designated Contracting States:
**AT BE CH CY DE DK ES FI FR GB GR IE IT LI LU MC NL PT SE TR**

(30) Priority: **07.03.2000 FI 20000515**

(43) Date of publication of application:
**04.12.2002 Bulletin 2002/49**

(73) Proprietor: **Vivoxid Oy**
**20520 Turku (FI)**

(72) Inventors:
• **Itälä, Ari**
**20520 Turku (FI)**
• **Aro, Hannu**
**20810 Turku (FI)**

• **Hupa, Mikko**
**20720 Turku (FI)**
• **Nordström, Egon**
**21600 Pargas (FI)**
• **Ylänen, Heimo**
**20810 Turku (FI)**

(74) Representative: **Heikkilä, Hannes Antero**
**Turun Patenttitoimisto Oy,**
**P.O. Box 99**
**20521 Turku (FI)**

(56) References cited:
**WO-A1-96/21628          WO-A1-97/06896**
**US-A- 4 871 384          US-A- 4 946 546**
**US-A- 5 232 878**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The invention relates to a method for etching the surface of a bioactive glass.

BACKGROUND OF THE INVENTION AND THE STATE OF THE ART

The publications to which reference is made and which are used for illustrating the background of the invention and the state of the art are to be deemed as being incorporated into the description of the invention below.

**Biomaterials and their biologic attachment**

Bioactive glass bonding chemically with bone has already been investigated for more than ten years (Andersson, Karlsson 1988).

Implants for both medical and dental purposes have long been prepared from a variety of materials. Various metals, metal alloys, plastics, ceramic materials, glass ceramic materials, and the latest, i.e. bioactive glasses, differ from one another not only by their durability but also by the properties of the interface between the implant and the tissue. Inert materials, such as metals and plastics, do not react with a tissue, in which case there always remains an interface between the implant and the tissue; the implant and the tissue constitute two distinct systems. Bioactive materials, such as hydroxyapatite, glass ceramic materials and bioactive glasses, react chemically with the tissue, whereupon there forms at the interface between the implant and the tissue a chemical bond, which is relatively strong, especially with bioactive glasses. The implant and the tissue are thus fixed to each other. The speed of the healing of the tissue and the possible chemical bond with the implant depend on the tissue activity of the implant material used.

International patent publication WO 96/21628, Brink *et al*., describes a group of bioactive glasses that can be processed easily. From such bioactive glasses it is possible, for example to draw fibers and, for example by the torch spraying technique, to prepare so-called microspheres of glass. Porous bioactive pieces are prepared by sintering these microspheres together. By using microspheres that are within as narrow a fraction as possible, the porosity of the body can be controlled. According to the literature it seems that the most advantageous particle size is within the fraction 200-400 microns (Schepers, Ducheyne 1997, Tsuruga *et al*. 1997, Schliephake *et al*. 1991, Higashi, Okamoto 1996). The studies carried out by the inventors so far have shown that porous bioactive implants prepared by sintering (sphere diameter 250-300 µm) have very strong new bone growth inducing action in the femur of a rabbit (Ylänen *et al*. 1997). The shear strength of the bioactive implants in a push-out to failure test has, already after three weeks *in vivo*, been statistically as high as after 12 weeks. The amount of bone inside the porous glass matrix has, after 12 weeks *in vivo*, been significantly higher than in a corresponding titanium implant. It is, however, advisable to note that in a bioactive matrix porosity increases steadily as a function of time as the bioactive glass mass is replaced by new bone. Porosity increased in experiments *in vivo* from 30 % to 65 %. In a titanium matrix, of course, porosity does not change. Thus the amount of new bone inside bioactive implants is *de facto* almost double that inside titanium implants. In our opinion this shows that the porous implant type used by us is right.

The beginning of new bone growth seems to be located in micro-cracks in the bioactive glass particles (Schepers, Ducheyne 1997). Evidently the calcium and phosphate dissolving from the glass into the fluid *(in vitro* SBF, *in vivo* intercellular fluid) surrounding the micro-crack rapidly form, together with the calcium and phosphate normally present in the fluid, so high a concentration that the solubility product of the ions concerned is exceeded. As a consequence of this, calcium phosphate precipitates onto the silica gel on the surface of the bioactive glass and new bone growth begins. The porous body sintered from bioactive microspheres is full of microscopically small cavities. This explains the rapid bone growth inducing property of the bodies we sintered from bioactive microspheres. According to the literature, the proteins most effectively controlling bone growth attach to the surface of bioactive glass (Ohgushi *et al*. 1993, Vrouwenvelder *et al*. 1992, Lobel, Hench 1998, Vrouwenvelder *et al*. 1993, Shimizu *et al*. 1997, Miller *et al*. 1991).

It has been shown that not only the biomaterial itself but also the correct roughness of the microparticle surfaces has a favorable effect on the attachment of the said proteins to the biomaterial surface (Grossner-Schreiber, Tuan 1991, Boyan *et al*. 1998).

The literature defines micro-roughness as surface roughness of a magnitude of <50 µm (Wen *et al*. 1996). On titanium surfaces it has been observed that a roughness of a magnitude of 10-50 µm has a favorable effect on the mechanical properties of the bone-implant interface, such as transmission of loads, mechanical attachment (Ratner 1983, Baro *et al*. 1986,), whereas a roughness of a magnitude of 10 nm -10 µm has a favorable effect on the attachment of the implant to bone through a normal healing process (Kasemo 1983). The fact that this roughness is in the order of magnitude of cells and large biomolecules facilitates the adsorption of cells and biomolecules to the said surface.

It has been shown that with a rough surface a more rapid early adhesion of cells and biomaterials (Kasemo 1983) and a higher bone bonding strength (Buser *et al*. 1998) are achieved than with a smooth surface. Above all, a rough surface also provides a larger surface for the attachment of cells than does a smooth surface.

An interesting observation was reported by Thomas *et al*. 1985, who observed in a histological study that direct apposition of bone was achieved with a rough surface of a prosthesis, whereas on the interface between a smooth-surfaced prosthesis and bone there a thin connective tissue layer was observed (Thomas *et al*. 1985). Several studies corroborate that fibroblasts (cells forming connective tissue) attach more weakly to rough surfaces (Kononen et al. 1992), whereas osteoblasts (cells forming bone tissue) attach more effectively to rough surfaces than to smooth surfaces (Michaels *et al*. 1989, Bowers *et al*. 1992).

In an *in vivo* investigation, an approx. 50 % larger bone-implant contact surface was attained at 3 weeks with a sand-blasted and etched titanium surface ("micro-roughest") than with a rough titanium surface coated by the plasma blasting technique (Buser 1998).

On the basis of the literature it is thus conceivable that the most optimal way of achieving rapid and maximally complete new bone growth inside a porous biomaterial is to use:

- a bioactive glass as the material
- a piece sintered from microparticles of 200-400 μm (preferably hollow microparticles or microparticles provided with depressions or thoroughgoing holes)
- totally etched microparticle surfaces.

A piece such as this would be not only in the microsize (etching in the microparticles) but also in the macrosize (microparticles sintered together form a porous entity) full of independent islands favorable for new bone growth. Etching would further accelerate the beginning of reactions necessary for the formation of new bone.

From a processable bioactive glass it is also possible to draw fibers, and from these fibers it is possible, potentially together with other fibers, to prepare a textile product, such as a felt, a fabric or a mat. The textile product can be used as an implant, for tissue control, as filler in bone cavities or soft tissue, etc.

**Etching, i.e. roughening, of a glass surface**

The glass phase can be examined as a three-dimensional network made up of $SiO_4$ tetrads. There are positive ions ($Na^+$, $Ca^{2+}$, etc.) in the network of the glass structure. In the etching or roughening of the glass surface, the $H^+$ ions of the solution and the positive ions of the glass are exchanged.

A sufficient acid attack is ensured if the hydrogen ion concentration in the solution is maintained at a sufficiently high level. Hydrofluoric acid reacts with the $SiO_2$ in the glass surface and forms silicon fluorides.

A mixture of hydrogen fluoride and a strong acid, such as sulfuric acid, is most commonly used for the etching of ordinary glass, i.e. window glass, and the pH of the etching process is very low, i.e. approx. 1.

In the etching of a bioactive glass surface by conventional means, i.e. with a mixture of hydrogen fluoride and a strong acid, the pH being approx. 1, the result has been a uniform gelling of the surface and not the desired point-like etching. This may be a result of the difference in the compositions of bioactive glass and ordinary glass. Ordinary glass differs in its composition from bioactive glass especially in that its $SiO_2$ content is typically above 60 %, whereas the $SiO_2$ content of bioactive glass is lower, i.e. clearly below 60 %. The CaO and MgO contents of bioactive glass, on the other hand, are higher than those of ordinary glass.

No suitable method has so far been presented in the literature for the etching of a bioactive glass surface.

OBJECT OF THE INVENTION AND SUMMARY OF THE INVENTION

The object of the present invention is to provide an etching or roughening method suitable for the surface of bioactive glass. It is a particular object to provide a method as a consequence of which there is obtained in the surface a point-like etching trace and not uniform etching or gelling of the surface. It is also an object to provide an etching method wherein the precipitation, on the glass surface, of poorly soluble compounds formed by the ions dissolved in consequence to the etching is prevented.

These objects are achieved by the method according to the invention, the characteristics of which are given in the claims.

The invention thus relates to a method for etching the surface of glass, wherein the glass surface is contacted with an acid fluoride solution. The method is characterized in that the glass is a bioactive glass and that the solution contains a complexing agent forming a complex with the ions dissolving from the glass.

DETAILED DESCRIPTION OF THE INVENTION AND PREFERRED EMBODIMENTS

By etching is meant in the present invention irregularities caused in a glass surface, the depth of the irregularities ranging from 10 nm to 50 μm, preferably within the region from 1 to 50 μm.

In the context of the definition of the present invention, by bioactive glass is meant a glass which in physiological conditions dissolves at least partly in a few months, preferably within a few weeks, most preferably within approximately 6 weeks. The $SiO_2$ content of bioactive glass is below 60 %.

The problem with many conventional bioactive glasses is that their processability is poor, since they tend to crystallize. It is not possible to manufacture spheres, fibers or other formed pieces from such bioactive glasses.

International patent application WO 96/21628 describes bioactive glasses of a novel type, the working range of which is suitable for the processing of glass and from which it is thus possible to manufacture spheres, fibers or other formed pieces, such as cylinders. The bioactive glasses described in this publication are also especially good for the reason that the processability of the glass has been achieved without the adding of aluminum oxide. Such glasses typically have the following composition:

| | |
|---|---|
| $SiO_2$ | 53 - 60 % by weight |
| $Na_2O$ | 0 - 34 % by weight |
| $K_2O$ | 1 - 20 % by weight |
| $MgO$ | 0 - 5 % by weight |
| $CaO$ | 5 - 25 % by weight |
| $B_2O_3$ | 0 - 4 % by weight |
| $P_2O_5$ | 0.5 - 6 % by weight |

However so that

$$Na_2O + K_2O = 16 - 35 \text{ \% by weight}$$

$$K_2O + MgO = 5 - 20 \text{ \% by weight}$$

and

$$MgO + CaO = 10 - 25 \text{ \% by weight.}$$

According to an especially preferred embodiment, the bioactive glass spheres, fibers or other pieces are made from a bioactive glass the composition of which is $Na_2O$ 6 % by weight, $K_2O$ 12 % by weight, $MgO$ 5 % by weight, $CaO$ 20 % by weight, $P_2O_5$ 4 % by weight and $SiO_2$ 53 % by weight.

Other especially suitable glass compositions include $Na_2O$ 6 % by weight, $K_2O$ 11 % by weight, $MgO$ 5 % by weight, $CaO$ 22 % by weight, $P_2O_5$ 2 % by weight, $SiO_2$ 53 % by weight and $B_2O_3$ 1 % by weight, as well as $Na_2O$ 4 % by weight, $K_2O$ 9 % by weight, $MgO$ 5 % by weight, $CaO$ 22 % by weight, $P_2O_5$ 4 % by weight, $SiO_2$ 55 % by weight and $B_2O_3$ 1 % by weight.

The acid fluoride solution suitable for the etching method according to the invention has a considerably high concentration of fluoride ions. This is achieved by using a fluoride compound having as high a water-solubility as possible. A good example to be mentioned is saturated aqueous solution of ammonium fluoride, having an ammonium fluoride concentration of approx. 27 M.

It has proven to be important in the etching of the surface of a bioactive glass that the acidity of the solution should not be too strong. Too low a pH causes uniform dissolving and gelling of the surface. A suitable pH is within the range 1.5 - 5, preferably 2 - 5. For this reason, acids too strong are to be avoided. One example of a highly suitable acid is citric acid. Citric acid is a quadribasic acid $H_4L$, the first ligand $H_3L^-$ of which is prevalent within the pH range 3.1- 4.7. In addition to citric acid providing a suitable pH level, there is a considerable advantage also in that the said ligand is capable of binding ions, in particular calcium ions, released from the glass surface and to form a water-soluble complex with them. As a result of this, the formation and precipitation on the glass surface of insoluble calcium compounds (in particular calcium fluoride and calcium phosphates) is prevented.

The chemical processes possibly occurring in the etching are depicted in the diagram. Owing to the hydrogen ions, silicon dissolves from the surface of the bioactive glass sphere. Silicon ions together with the fluoride ions form a water-soluble compound $SiF_6^{2-}$. Calcium ions are also released, and they can form a water-soluble complex together with the citric acid ligand $H_3L^-$. This prevents the formation of poorly soluble Ca compounds and their precipitation on the surface of the etched glass. Such poorly soluble Ca compounds include Ca phosphate and $CaF_2$.

The anion of the acid used in the etching need not necessarily at the same time act as a complexing agent.

It is also conceivable that the complexing agent is added as a separate component.

If the roughening or etching solution is based on ammonium fluoride and citric acid, the etching solution contains citric acid suitably 5 - 80 % by volume. The concentration of the citric acid used for the preparation of the etching solution is either a saturated solution, i.e. 8.5 M (163 g of citric acid/100 ml of water), or more dilute. The ammonium fluoride solution used for the etching solution is either a saturated solution (27 M; 100 g/100 ml of water) or more dilute.

The time required for the etching depends, among other things, on the concentration of the etching solution, the temperature, the composition of the glass, etc., and ranges from a few seconds to up to several hours.

According to a preferred embodiment, spheres or other pieces, such as cylinders, are formed from the bioactive glass. These bioactive particles are sintered together, either as such or possibly with other, weakly bioactive or non-bioactive particles to form a porous composite. A weakly bioactive or non-bioactive glass is one that does not dissolve in physiological conditions within the first months. The preferable diameter of the particles is approx. 200 - 400 $\mu$m. Preferably, the composite is a piece sintered from hollow particles or from particles provided with depressions or throughgoing holes. One suitable particle size that can be mentioned is a glass cylinder, which can be made, for example, by drawing from a bioactive glass a thin capillary tube, which is cut into short pieces by means of, for example, a carbonic acid laser. In connection with the cutting, the capillary tube may become blocked at one end or both ends, whereby a piece provided with a depression or a hollow piece is formed. If the capillary tube is not blocked, a piece provided with a thoroughgoing hole is obtained. This sintered composite is contacted with the etching solution, whereby a particle surface etched throughout is obtained.

A piece such as this is not only in the microsize (etching on the particle surfaces) but also in the macrosize (microparticles sintered together form a porous entity) full of independent islands favorable for new bone growth. Etching further speeds up the beginning of reactions necessary for the formation of new bone.

From a processable bioactive glass it is also possible to draw fibers, and from these fibers, possibly together with other fibers, it is possible to produce a textile product, such as a felt, fabric or mat. The textile product can be used as an implant, for tissue control, as a filler material in bone cavities or soft tissue, etc. This textile product is contacted with an etching solution, whereby fiber surfaces etched throughout are obtained. The etching of the fiber surfaces provides advantageous results corresponding to those achieved through the etching of particle surfaces.

According to one embodiment there is formed on the surfaces of particles or fibers one or several bioactive layers made up of, for example, silica gel and/or hydroxyapatite. Although it is possible to form such bioactive layers on the surfaces of smooth particles or fibers it is, however, advantageous first to etch the particle or fiber surfaces. Such pre-corrosion, i.e. forming of a bioactive layer, can be achieved by means of, for example, simulated body fluid (SBF) or some organic or inorganic solvent.

The method for etching a bioactive glass surface will be described in greater detail with the help of the following example.

EXAMPLE

Porous glass cones A, B and C (length 5 mm, diameters 3 mm and 3.2 mm) were made by sintering together glass spheres having diameters of 250...315 $\mu$m. Three different bioactive glasses 13-93 (Cone A), 3-98 (Cone B) and 1-98 (Cone C) were used. The compositions of the different bioactive glasses are
13-93: $Na_2O$ 6 % by weight, $K_2O$ 12 % by weight, MgO 5 % by weight, CaO 20 % by weight, $P_2O_5$ 4 % by weight, and $SiO_2$ 53 % by weight;
3-98: $Na_2O$ 4 % by weight, $K_2O$ 9 % by weight, MgO 5 % by weight, CaO 22 % by weight, $P_2O_5$ 4 % by weight, $SiO_2$ 55 % by weight, and $B_2O_3$ 1 % by weight; and
1-98: $Na_2O$ 6 % by weight, $K_2O$ 11 % by weight, MgO 5 % by weight, CaO 22 % by weight, $P_2O_5$ 2 % by weight, $SiO_2$ 53 % by weight, and $B_2O_3$ 1 % by weight.

The glass cones were immersed in a roughening or etching solution containing ammonium fluoride and citric acid. Aqueous solutions of ammonium fluoride (22 M) and citric acid (8.5 M) were prepared separately, whereafter they were combined, undiluted, at the ratio
Ammonium fluoride solution : citric acid solution = 1:3
The etching periods were: Cone A 15 min, Cone B 20 min, and Cone C 2 min.

The etching was discontinued by immersing the cones in distilled water, whereby the etching solution was removed by rinsing from the glass surface. Thereafter the water (corroding the glass surface) was removed by immersing the cones in ethanol.

Any salt deposits were removed from the glass surface by an ultrasound wash by immersing the cones in a 1.2 M hydrochloric acid. The treatment periods were: Cone A 35 s, Cone B 55 s, and Cone C 20 s. The hydrochloric acid wash was discontinued by immersing the cones in distilled water, and the water was removed by immersing them in ethanol.

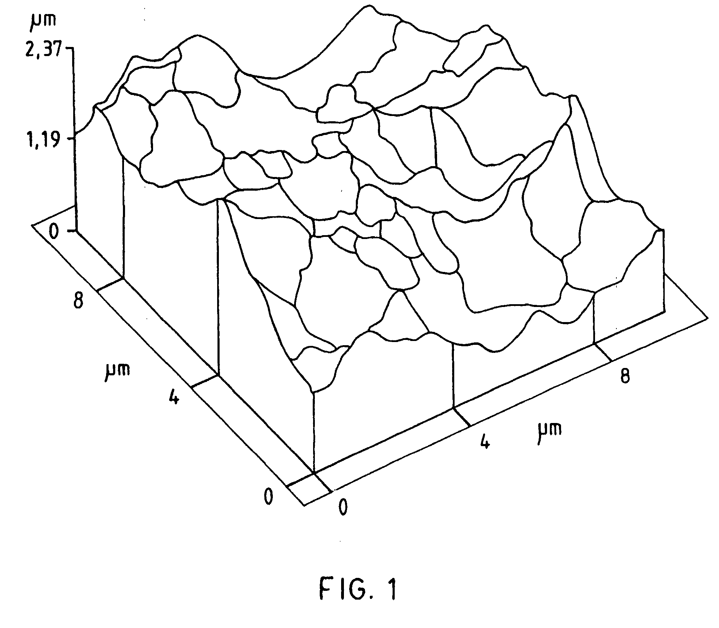

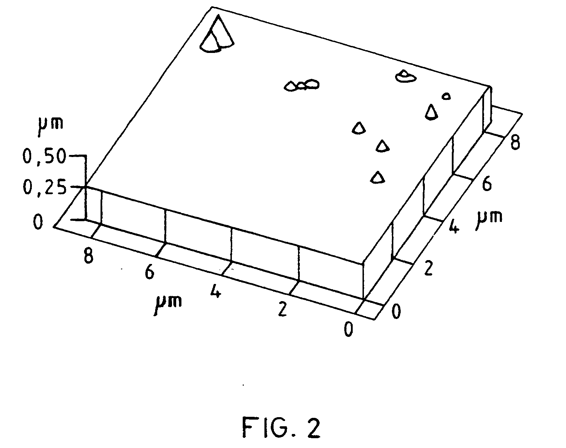

Figure 1 shows an atomic force micrograph (AFM, Atomic Force Microscopy) of the surface of Cone A after the etching. Figure 2 shows a corresponding micrograph of the surface of Cone A before the etching (control).

The above-mentioned embodiments of the invention are only examples of the implementation of the idea according to the invention. For a person skilled in the art it is clear that the various embodiments of the invention may vary within the scope of the claims presented below.

References

Andersson ÖH, Karlsson KH: Models for physical properties and bioactivity of phosphate opal glasses. Glastech Ber 61:200-305, 1988.

Schepers EJ, Ducheyne P: Bioactive glass particles of narrow size range for the treatment of oral bone defects: a 1-24 month experiment with several materials and particle sizes and size ranges. J. Oral Rehabil, 24(3):171-181, (1997).

Tsuruga E, Takita H, Itoh H, Wakisaka Y, Kuboki Y: Pore size of porous hydroxyapatite as the cell-substratum controls BMP-induced osteogenesis. J Biochem (Tokyo) 121(2):317-324, (1997).

Schiephake H, Neukam FW, Klosa D: Influence of pore dimension on bone ingrowth into porous hydroxylapatite blocks used as bone graft substitutes. A histometric study. Int J Oral Maxillofac Surg 20(1):53-58, (1991).

Higashi T, Okamoto H: Influence of particle size of hydroxyapatite as a capping agent on cell proliferation of cultured fibroblasts. J Endod 22(5):236-239, (1996).

Ylänen H, Karlsson KH, Heikkilä JT, Mattila K, Aro HT: 10th International Symposium on Ceramics in Medicine, Paris, (1997).

Grossner-Schreiber B, Tuan RS: The influence of the titanium implant surface on the process of osseointegration. Dtsch Zahnartzl Z 46(10):691-693, (1991).

Boyan BD, Batzer R, Kieswetter K, Liu Y, Cochran DL, Szmuckler-Moncler S, Dean DD, Schwartz Z: Titanium surface roughness alters responsiveness of MG63 osteoblast-like cells to alpha,25-(OH)2D3. J Biomed Mater Res 39(1):77-85, (1998).

Ohgushi H, Dohi Y, Tamai S, Tabata S: Osteogenic differentiation of marrow stromal stem cells in porous hydroxyapatite ceramics. J Biomed Mater Res 27(11):1401-1407, (1993).

Vrouwenvelder WC, Groot CG, de Groot K: Behaviour of fetal rat osteoblasts cultured in vitro on bioactive glass and nonreactive glasses. Biomaterials 13(6):382-392, (1992).

Lobel KD, Hench LL: In vitro adsorption and activity of enzymes on reaction layers of bioactive glass substrates. J Biomed Mater Res 39(4):575-579, (1998).

Vrouwenvelder WC, Groot CG, de Groot: Histological and biochemical evaluation of osteoblasts cultured on bioactive glass, hydroxylapatite, titanium alloy, and stainless steel. J Biomed Mater Res 27(4):465-475, (1993).

Shimizu Y, Sugawara H, Furusawa T, Mizunuma K, Inada K, Yamashita S: Bone remodeling with resorbable bioactive glass and hydroxyapatite. Implant Dent 6(4):269-274, (1997).

Miller TA, Ishida K, Kobayashi M, Wollman JS, Turk AE, Holmes RE: The induction of bone by an osteogenic protein and the conduction of bone by porous hydroxyapatite: a laboratory study in the rabbit. Plast Reconstr Surg 87(1):87-95, (1991).

Kasemo B: Biocompatibity of titanium implants: surface science aspects, J. Prosth. Dent.49 (1983),832-837.

Ratner BD: Surface characterization of biomaterials by electron spectroscopy for chemical analysis. Ann Biomed. Eng. 11 11 (1983) 313-336.

Baro AM, Garcia N, Miranda et al. Characterization of surface roughness in titanium dental implants measured with scanning tunneling microscopy at athmospheric pressure, Biomaterials 7 (1986), 463-466.

Buser D, Nydegger T, Oxland T, Cochran DL, Schenk RK, Hirt HP, Snétivy D, Nolte LP. Interface shear strength of titanium implants with a sandblasted and acid-etched surface. A biomechanical study in the maxilla of miniature pigs.

Thomas KA, Cook SD. An evaluation of variables influencing implant fixation by direct bone apposition. J. Biomed. Mater. Res 1985 19:875-901.

Michaels C, Keller J, Stanford C, Solursh M. In vitro cell attachment of osteoblast-like cells to titanium. J Dent Res 1989; 68:276-28 1.

Bowers KT, Keller J, Randolph BA, et al. Optimization of surface micromorphology for enhanced osteoblast responses in vitro. Int J Oral maxillofacial Implants 1992; 7:302.310.

Kononen M, Hormia M, Kivilahti J et al. Effects of surface processing on the attachment, orientation, and proliferation of human gingival fibroblasts on titanium. J.Biomed. Mater. Res. 26(1992), 1325-1337.

Wen X, Wang X, Zhang N. Microrough surface of metallic biomaterials: literature review. Bio. Med. Mat. Eng. 6 (1996) 173-189.

Blonder GE, Johnson BH. Wet chemical etching technique for optical fibers. U.S. Pat. No. 5,200,024.

Carlsson R. Korrosion av glasyrer. Svenska Keramiska Sällskapets kurs 1999; Lektion 9: 1-15.

**Claims**

1. A method for etching the surface of glass, wherein the glass surface is contacted with an acid fluoride solution, **characterized in that** the glass is a bioactive glass and that the solution contains a complexing agent which forms a complex with the ions dissolving from the glass, and that the pH of the solution is within the range 1.5 - 5.

**2.** The method according to claim 1, **characterized in that** the pH of the solution is within the range 2 - 5.

**3.** The method according to claim 1 or 2, **characterized in that** the desired acidity of the solution is achieved with an acid which at the same time serves as a complexing agent.

**4.** The method according to claim 3, **characterized in that** the acid is citric acid.

**5.** The method according to any of claims 1 - 4, **characterized in that** the bioactive glass is easily processable, and that fibers, spheres, cylinders, or pieces coated with the said glass, or other objects are made from the said bioactive glass.

**6.** The method according to claim 5, **characterized in that** the composition of the bioactive glass is

| | |
|---|---|
| $SiO_2$ | 53 - 60 % by weight |
| $Na_2O$ | 0 - 34 % by weight |
| $K_2O$ | 1 - 20 % by weight |
| $MgO$ | 0 - 5 % by weight |
| $CaO$ | 5 - 25 % by weight |
| $B_2O_3$ | 0 - 4 % by weight |
| $P_2O_5$ | 0.5 - 6 % by weight |

however so that

$$Na_2O + K_2O = 16 - 35 \text{ % by weight}$$

$$K_2O + MgO = 5 - 20 \text{ % by weight,}$$

and

$$MgO + CaO = 10 - 25 \text{ % by weight.}$$

**7.** The method according to claim 6, **characterized in that** the composition of the bioactive glass is
$Na_2O$ 6 % by weight, $K_2O$ 12 % by weight, $MgO$ 5 % by weight, $CaO$ 20 % by weight, $P_2O_5$ 4 % by weight and $SiO_2$ 53 % by weight, or
$Na_2O$ 6 % by weight, $K_2O$ 11 % by weight, $MgO$ 5 % by weight, $CaO$ 22 % by weight, $P_2O_5$ 2 % by weight, $SiO_2$ 53 % by weight and $B_2O_3$ 1 % by weight, or
$Na_2O$ 4 % by weight, $K_2O$ 9 % by weight, $MgO$ 5 % by weight, $CaO$ 22 % by weight, $P_2O_5$ 4 % by weight, $SiO_2$ 55 % by weight, and $B_2O_3$ 1 % by weight.

**8.** The method according to claim 5, 6 or 7, **characterized in that** the bioactive glass spheres or cylinders are sintered together, possibly together with non-bioactive or weakly bioactive glass spheres or cylinders, to form a porous composite, which is contacted with the etching solution.

**9.** The method according to claim 5, 6 or 7, **characterized in that** from the bioactive fibers, possibly together with weakly bioactive glass fibers, there is formed a porous textile product, which is contacted with the etching solution.

**10.** The method according to any of the preceding claims, **characterized in that** a bioactive calcium phosphate layer is formed on the etched glass surface.

**Patentansprüche**

**1.** Verfahren zum Ätzen der Oberfläche von Glas, wobei sich die Glasoberfläche in Kontakt mit einer sauren Fluoridlösung befindet, **dadurch gekennzeichnet, dass** das Glas ein bioaktives Glas ist und dass die Lösung einen Komplexbildner enthält, der mit den Ionen, die aus dem Glas herausgelöst werden, einen Komplex bildet, und dass der pH-Wert der Lösung innerhalb des Bereichs von 1,5-5 liegt.

2. Verfahren nach Anspruch 1, **dadurch gekennzeichnet, dass** der pH-Wert der Lösung innerhalb des Bereichs von 2-5 liegt.

3. Verfahren nach Anspruch 1 oder 2, **dadurch gekennzeichnet, dass** die gewünschte Acidität der Lösung mit einer Säure erzielt wird, die gleichzeitig als ein Komplexbildner dient.

4. Verfahren nach Anspruch 3, **dadurch gekennzeichnet, dass** die Säure Zitronensäure ist.

5. Verfahren nach einem der Ansprüche 1-4, **dadurch gekennzeichnet, dass** das bioaktive Glas leicht verarbeitbar ist und dass Fasern, Kugeln, Zylinder oder Stücke, die mit diesem Glas überzogen sind, oder andere Gegenstände aus dem bioaktiven Glas hergestellt werden.

6. Verfahren nach Anspruch 5, **dadurch gekennzeichnet, dass** die Zusammensetzung des bioaktiven Glases wie folgt lautet:

| | |
|---|---|
| $SiO_2$ | 53-60 Gew.-% |
| $Na_2O$ | 0-34 Gew.-% |
| $K_2O$ | 1-20 Gew.-% |
| MgO | 0-5 Gew.-% |
| CaO | 5-25 Gew.-% |
| $B_2O_3$ | 0-4 Gew.-% |
| $P_2O_5$ | 0.5-6 Gew.-% |

so, dass

$$Na_2O + K_2O = 16\text{-}35 \text{ Gew.-\%}$$

$$K_2O + MgO = 5\text{-}20 \text{ Gew.-\%},$$

und

$$MgO + CaO = 10\text{-}25 \text{ Gew.-\%}.$$

7. Verfahren nach Anspruch 6, **dadurch gekennzeichnet, dass** die Zusammensetzung des bioaktiven Glases 6 Gew.-% $Na_2O$, 12 Gew.-% $K_2O$, 5 Gew.-% MgO, 20 Gew.-% CaO, 4 Gew.-% $P_2O_5$ und 53 Gew.-% $SiO_2$ oder 6 Gew.-% $Na_2O$, 11 Gew.-% $K_2O$, 5 Gew.-% MgO, 22 Gew.-% CaO, 2 Gew.-% $P_2O_5$, 53 Gew.-% $SiO_2$ und 1 Gew.-% $B_2O_3$ oder 4 Gew.-% $Na_2O$, 9 Gew.-% $K_2O$, 5 Gew.-% MgO, 22 Gew.-% CaO, 4 Gew.-% $P_2O_5$, 55 Gew.-% $SiO_2$ und 1 Gew.-% $B_2O_3$ ist.

8. Verfahren nach Anspruch 5, 6 oder 7, **dadurch gekennzeichnet, dass** die bioaktiven Glaskugeln oder -zylinder, möglicherweise zusammen mit nicht-bioaktiven oder schwach bioaktiven Glaskugeln oder -zylindern, unter Bildung eines porösen Verbundstoffes, der mit der Ätzlösung in Kontakt gebracht wird, zusammengesintert werden.

9. Verfahren nach Anspruch 5, 6 oder 7, **dadurch gekennzeichnet, dass** aus den bioaktiven Fasern, möglicherweise zusammen mit den schwach bioaktiven Glasfasern, ein poröses Textilprodukt gebildet wird, das mit der Ätzlösung in Kontakt gebracht wird.

10. Verfahren nach einem der voranstehenden Ansprüche, **dadurch gekennzeichnet, dass** auf der geätzten Glasoberfläche eine bioaktive Calciumphosphatschicht gebildet wird.

**Revendications**

1. Procédé pour graver une surface de verre, dans lequel la surface de verre est mise en contact avec une solution de fluorure acide, **caractérisé en ce que** le verre est un verre bioactif et **en ce que** la solution contient un agent complexant qui forme un complexe avec les ions se dissolvant provenant du verre, et **en ce que** le pH de la solution se situe dans la gamme de 1,5 à 5.

2. Procédé selon la revendication 1, **caractérisé en ce que** le pH de la solution se situe dans la gamme de 2 à 5.

3. Procédé selon la revendication 1 ou 2, **caractérisé en ce que** l'acidité souhaitée de la solution est obtenue à l'aide d'un acide qui, en même temps, sert d'agent complexant.

4. Procédé selon la revendication 3, **caractérisé en ce que** l'acide est l'acide citrique.

5. Procédé selon l'une quelconque des revendications 1 à 4, **caractérisé en ce que** le verre bioactif peut facilement être transformé, et **en ce que** des fibres, des sphères, des cylindres ou des pièces revêtues dudit verre ou d'autres objets sont faits à partir dudit verre bioactif.

6. Procédé selon la revendication 5, **caractérisé en ce que** la composition du verre bioactif comprend :

| | |
|---|---|
| $SiO_2$ | 53% à 60% en poids |
| $Na_2O$ | 0% à 34% en poids |
| $K_2O$ | 1% à 20% en poids |
| MgO | 0% à 5% en poids |
| CaO | 5% à 25% en poids |
| $B_2O_3$ | 0% à 4% en poids |
| $P_2O_5$ | 0,5% à 6% en poids |

à condition, toutefois, que :

$$Na_2O + K_2O = 16\% \text{ à } 35\% \text{ en poids}$$

$$K_2O + MgO = 5\% \text{ à } 20\% \text{ en poids}$$

$$MgO + CaO = 10\% \text{ à } 25\% \text{ en poids}$$

7. Procédé selon la revendication 6, **caractérisé en ce que** la composition du verre bioactif comprend :

6% en poids de $Na_2O$, 12% en poids de $K_2O$, 5% en poids de MgO, 20% en poids de CaO, 4% en poids de $P_2O_5$ et 53% en poids de $SiO_2$, ou

6% en poids de $Na_2O$, 11% en poids de $K_2O$, 5% en poids de MgO, 22% en poids de CaO, 2% en poids de $P_2O_5$, 53% en poids de $SiO_2$ et 1% en poids de $B_2O_3$, ou

4% en poids de $Na_2O$, 9% en poids de $K_2O$, 5% en poids de MgO, 22% en poids de CaO, 4% en poids de $P_2O_5$, 55% en poids de $SiO_2$ et 1% en poids de $B_2O_3$.

8. Procédé selon la revendication 5, 6 ou 7, **caractérisé en ce que** les sphères ou les cylindres de verre bioactif sont frittés ensemble, éventuellement conjointement avec des sphères ou des cylindres de verre non bioactif ou faiblement bioactif, pour former un composite poreux, qui est mis en contact avec la solution de gravure.

9. Procédé selon la revendication 5, 6 ou 7, **caractérisé en ce que**, à partir des fibres bioactives, éventuellement conjointement avec des fibres de verre faiblement bioactives, il se forme un produit textile poreux, qui est mis en contact avec la solution de gravure.

10. Procédé selon l'une quelconque des revendications précédentes, **caractérisé en ce qu'**une couche de phosphate de calcium bioactif est formée sur la surface de verre gravée.

FIG. 1

FIG. 2